# EUROPÄISCHE PATENTSCHRIFT

(11) **EP 1 256 325 B1**
(45) Veröffentlichungstag und Bekanntmachung des Hinweises auf die Patenterteilung: **27.04.2005**
(21) Anmeldenummer: 02003785.9
(22) Anmeldetag: 20.02.2002
(51) Int. Cl.: A61B 18/14

(54) **Urologisches Resektoskop mit Isolierkörper**
Urological resectoscope with insulating mantel
Resectoscope pour urologie avec manteau isolant

(30) Priorität: 09.05.2001 DE 10122465
(43) Veröffentlichungstag der Anmeldung: 13.11.2002
(73) Patentinhaber: OLYMPUS WINTER & IBE GMBH, 22045 Hamburg (DE)
(72) Erfinder: Valencic, Maksim, Dr., 51211 Matulji (HR); Nussbaum, Felix, 22089 Hamburg (DE); Wosnitza, Thomas, 21337 Lüneburg (DE)
(74) Vertreter: Schaefer, Konrad, Dipl.-Phys.

(56) Entgegenhaltungen:
- DE-A- 2 502 863
- DE-A- 2 617 556
- DE-U- 7 626 244
- US-A- 4 506 668
- US-A- 5 088 998

## Beschreibung

Die Erfindung betrifft ein urologisches Resektoskop der im Oberbegriff des Anspruches 1 genannten Art.

Ein nicht gattungsgemäßes Resektoskop ergibt sich aus DE 2428000, Figur 8. Diese Schrift stammt von Dr. Iglesias, der damit das erste Dauerspülresektoskop vorlegte. Durch die Doppelschaftausbildung werden getrennte Zufluß- und Abflußkanäle für die Spülung des Gesichtsfeldes sichergestellt, so daß langzeitig unter guter Sicht resiziert werden kann.

Bei solchen Resektoskopen wird zum Resizieren die Schneidschlinge zunächst distal ausgefahren und sodann, durch Gewebe schneidend, zurückgezogen. Zum sauberen Abschneiden der Gewebestreifen muß die Schneidschlinge gegen den distalen Schaftrand, der eine Schneidkante bildet, abschneiden. Dabei kommt es zu hoher Wärmebelastung des Schaftes. Dieser distale Randbereich des Schaftes muß also aus hochwarmfestem Material bestehen, das zudem elektrisch isolierend ist, um HF-Kontakt zu dem das Resektoskop haltenden Operateur zu vermeiden.

Bei der in der oben genannten Literaturstelle beschriebenen Konstruktion besteht ausweislich der Schraffur (nach US-Norm) der Außenschaft, der die Schneidkante für die Schneidschlinge ausbildet, aus nichtleitendem Material. Den gesamten Außenschaft aus Kunststoff oder Keramik auszubilden, bringt jedoch erhebliche Kosten- und Handhabungsnachteile mit sich.

Die DE 26 17 556 C2, ebenfalls von Dr. Iglesias, zeigt eine nicht gattungsgemäße Konstruktion, bei der der Außenschaft, der Innenschaft und der Isolierkörper fest miteinander zu einer Baueinheit verbunden sind, die als Ganzes vom Hauptkörper des Resektoskopes abnehmbar sind. Damit werden komplizierte Konstruktionsprobleme im Bereich des Isolierkörpers umgangen. Es ergibt sich jedoch eine Konstruktion, bei der der zur Ableitung von Flüssigkeit dienende Ringraum zwischen Innenschaft und Außenschaft nur sehr schlecht bzw. überhaupt nicht reinigbar ist. Unter modernen Sterilitätsgesichtspunkten ist diese Konstruktion somit völlig unbrauchbar. Der Isolierkörper ist außerdem bei dieser Konstruktion relativ dickwandig.

Mit dem DE-U-74 26 959 wurde eine Konstruktion vorgelegt, bei der beide Schäfte aus Metall bestehen und ein gesonderter Isolierkörper, der die Schneidkante ausbildet, vorgesehen ist. Der Isolierkörper ist bei dieser Konstruktion am Innenschaft befestigt. Diese Konstruktionsweise entwickelte sich zum heutigen Standard.

Diese Konstruktion weist einen geringfügigen, für den medizinischen Einsatz jedoch erheblichen Nachteil auf.

Der rohrförmige Isolierkörper bildet das distale Endstück des Innenschaftes. Wegen des notwendigen Ringraumes zwischen den Schäften hat der Außenschaft einen größeren Durchmesser als der Isolierkörper. Der Isolierkörper bestimmt jedoch mit seinem Innendurchmesser die mögliche Größe der Schneidschlinge. Diese Bauart bedingt somit einen Außendurchmesser, der erheblich größer ist als der Durchmesser der Schneidschlinge.

Der Operateur wünscht sich eine möglichst große Schneidschlinge, um zeitsparend möglichst großvolumige Gewebestreifen abschneiden zu können. Andererseits wünscht er sich ein Resektoskop mit möglichst geringem Außendurchmesser, um den Gewebekanal, durch den das Resektoskop eingeführt wird, üblicherweise die menschliche Urethra, nicht zu stark zu dehnen und damit durch Geweberisse zu traumatisieren. Die Durchmesserdifferenz zwischen Innendurchmesser des Isolierkörpers und Außendurchmesser des Außenschaftes soll also möglichst gering sein. Mit Resektoskopen nach dem Stand der Technik ergaben sich dabei jedoch die beschriebenen Probleme.

Aus der gattungsgemäßen DE-U-76 26 244 ist ein Resektoskop bekannt, bei dem der Außenschaft vom Innenschaft zu Reinigungszwecken lösbar ist. Der Isolierkörper ist am Außenschaft befestigt und hat einen Innendurchmesser, der kleiner ist als der Innendurchmesser des Außenschaftes. Diese Konstruktion kann jedoch das erwähnte Durchmesserproblem nicht lösen. Der Isolierkörper ist bereichsweise sehr dick ausgebildet, um in seinem Inneren einen den Ringraum verlängernden Kanal mit Löchern nach außen zu schaffen. Dadurch wird der für die Schneidschlinge zur Verfügung stehende Raum bereits verkleinert. Es kommt hinzu, dass der im Isolierkörper geschaffene Kanal nach Innen offen und dort von einem vorspringenden Bereich des Innenschaftes abgedeckt ist. Die übrigen Innenbereiche des Isolierkörpers werden von einer zusätzlichen, ihm innen anliegenden Kunststoffhülle abgedeckt. Dadurch wird zusätzlich der für die Schneidschlinge zur Verfügung stehende Raum verkleinert.

Die Aufgabe der vorliegenden Erfindung besteht darin, ein gattungsgemäßes Resektoskop zu schaffen, bei dem die Durchmesserdifferenz zwischen Innendurchmesser des Isolierkörpers und Außendurchmesser des Außenschaftes verringert werden kann.

Diese Aufgabe wird mit den Merkmalen des Anspruches 1 gelöst.

Erfindungsgemäß stößt der Innenschaft mit seinem aufgeweiteten distalen Rand gegen den proximalen Rand des Isolierkörpers. Er liegt diesem also nicht auf der Innenseite an und verringert nicht den zur Verfügung stehenden Innendurchmesser. Insbesondere kann dabei der Innenschaft mit seinem distalen Rand dem Innendurchmesser des Außenschaftes entsprechen. Die Wandstärke des Isolierkörpers kann daher auf ein Maß begrenzt werden, das der Summe der Wandstärken von Außenschaft und Innenschaft entspricht. Gegenüber handelsüblichen Resektoskopen kann dadurch der Außendurchmesser des Resektoskopes um etwa 1 mm reduziert werden bzw. bei gleichbleibendem Außendurchmesser der Schlingendurchmesser um etwa 1 mm vergrößert werden. Da der distale Rand des Innenschaftes gegen den proximalen Rand des Isolierkörpers stößt, lassen sich die Innenflächen von Isolierkörper und Innenschaft ohne Vorsprung glatt übergehend gestalten.

Vorteilhaft sind dabei die bereits aus der erstzitierten Druckschrift bekannten Merkmale des Anspruches 2 vorgesehen. Hierdurch wird erreicht, daß der Durchmesser der Schneidschlinge nicht durch den Innendurchmesser des Innenschaftes begrenzt wird.

Die Schneidschlinge wird in üblicher Konstruktion von am distalen Ende des Elektrodenträgers vorgesehenen Gabelarmen getragen, die möglichst großen Abstand haben sollen, um die Schneidschlinge stabil zu halten. Zur Schaffung eines großlumigen Rückstromkanales muß jedoch der Innendurchmesser des Innenschaftes klein sein. Zur Lösung dieses Problems sind vorteilhaft die Merkmale des Anspruches 3 vorgesehen. Auf diese Weise läßt sich mit einem kleinlumigen Innenschaft ein großlumiger Rückstromkanal schaffen, wobei dennoch die in nach außen gewölbten Nuten des Innenschaftes verlaufenden Gabelarme großen Abstand haben können.

Bei modernen Resektoskopen sind die beiden Schäfte an ihrer proximalen Befestigungsstelle drehbar aneinander gelagert, wie dies die DE 4101472 C2 zeigt. Beim ständigen Drehen des Resektoskopes, um unter unterschiedlichen Winkellagen schneiden zu können, wie dies insbesondere bei der Prostataresektion erforderlich ist, ergibt sich der Vorteil, daß der Außenschaft in der Urethra drehfest und somit besonders atraumatisch liegenbleiben kann, während Schneidschlinge und Innenschaft gedreht werden. Dies ergibt den weiteren Vorteil, daß bei Ausbildung des distalen Endbereiches der Schäfte gemäß DE-U-74 26 959 mit einem als abgeschrägter Schnabel ausgebildeten, am Innenschaft befestigten Isolierkörper die Schnabelform mit der Schneidschlinge dreht und somit für eine konstante Schneidkante gesorgt ist. Die abgeschrägte Schnabelform weist strömungsführende Vorteile auf.

Sitzt der Isolierkörper jedoch fest am Außenschaft und wird die Schneidschlinge gedreht, so kann zunächst nur eine gerade abgeschnittene distale Endkante des Isolierkörpers gewählt werden, da nur diese unter allen Drehrichtungen konstante Abschneidbedingungen gewährleistet. Um hier abzuhelfen sind vorteilhaft die Merkmale des Anspruches 4 vorgesehen. Hierbei sitzt der Isolierkörper zwar am Außenschaft, ist an diesem jedoch drehbar befestigt und mit dem Innenschaft drehgekoppelt. Beim Drehen des Innenschaftes mit der Schneidschlinge wird somit der Isolierkörper mitgedreht. Dies hat den Vorteil, daß der Isolierkörper nunmehr mit der gewünschten abgeschrägten Schnabelform versehen werden kann.

Vorzugsweise sind die Merkmale des Anspruches 5 vorgesehen. Ist der distale Rand des Innenschaftes schräg verlaufend angeordnet, so ergeben sich Bereiche des distalen Randes, die weiter distal liegen und Bereiche dieses Randes, die weiter proximal liegen. An dem weiter distal liegenden Bereichen reicht der zwischen Innenschaft und Außenschaft gebildete Ringraum weiter nach distal und es können dort Öffnungen vorgesehen sein, die sehr weit distal liegend Flüssigkeit ansaugen können. Die ist von Vorteil, wenn der Außenschaft des Resektoskopes bis nahe an sein distales Ende eng umschlossen ist. Es kann damit auch unter ungünstigen Bedingungen noch eine freie Dauerspülung sichergestellt werden. Die Abschrägung des distalen Randes des Innenschaftes ist vorteilhaft so gewählt, daß der Rand in dem Umfangsbereich in dem der mittlere Bereich der Schneidschlinge befindet, weiter proximal liegt und somit dort die Schneidschlinge ausreichend weit zurückgezogen werden kann und zwar bis in einen Bereich, an dem gegenüberliegend bereits Saugöffnungen liegen können.

In der Zeichnung ist die Erfindung beispielsweise und schematisch dargestellt. Es zeigen:
- Figur 1: eine teilgeschnittene Seitenansicht des Schaftbereiches eines erfindungsgemäßen Resektoskopes,
- Figur 2: eine distale Achsansicht des Resektoskopes der Figur 1,
- Figur 3: einen distalen Ausschnitt aus Figur 1 in einer Ausführungsvariante mit drehbarem Isolierkörper und
- Figur 4: in Darstellung gemäß Figur 3 eine Ausführungsvariante mit distal abgeschrägtem Innenschaft.

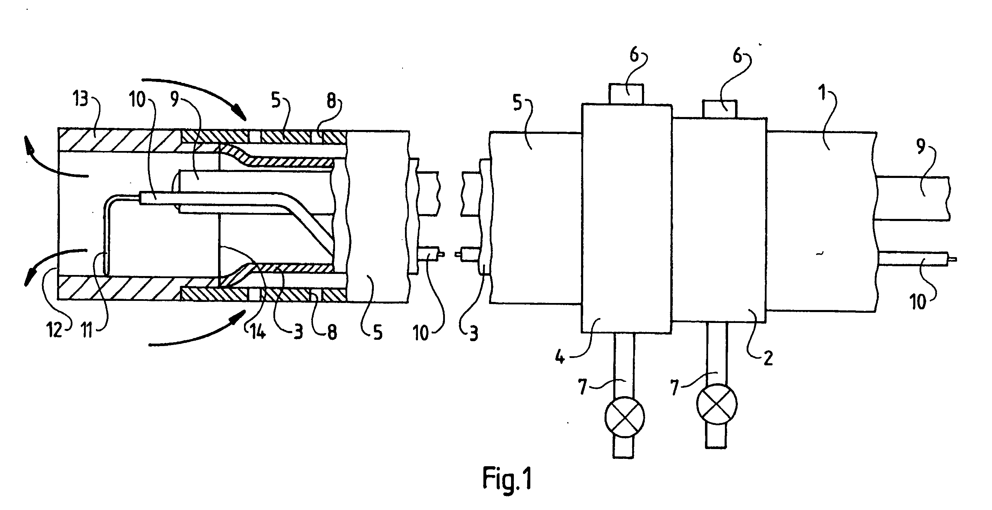

Figur 1 zeigt in teilgeschnittener Seitenansicht den Schaft eines Resektoskopes mit teilweise dargestelltem Hauptkörper 1, dessen wesentliche konstruktive Elemente wie Schlingentransporteur, Optikdurchführung, HF-Anschluß etc. zur zeichnerischen Vereinfachung weggelassen sind. Sie können z. B. entsprechend der eingangs zitierten Literaturstelle gestaltet sein.

An dem Hauptkörper 1 ist mit einem Kupplungsstück 2 ein Innenschaft 3 befestigt. Ferner ist mit einem Kupplungsstück 4 ein Außenschaft 5 befestigt. Die Kupplungsstücke 2, 4 lassen sich mit Arretiereinrichtungen 6 an- und abkuppeln, um den Schaftbereich zu Reinigungszwecken zerlegen zu können. An den Kupplungsstücken 2 und 4 sind Spülanschlüsse 7 vorgesehen zum Anschluß von Spülschläuchen, mit denen Spülflüssigkeit in das Innere des Innenschaftes 3 geführt werden kann und aus dem Ringraum zwischen Innenschaft 3 und Außenschaft 5 abgeführt werden kann. Es ergibt sich der gewünschte, mit Pfeilen dargestellte Strömungsverlauf um die distale Spitze des Schaftes, wobei Löcher 8 im Außenschaft 5 die rückströmende Flüssigkeit in den Ringraum einströmen lassen. Beide Schäfte 3, 5 sind in üblicher Weise als Metallrohr ausgebildet.

Im Inneren des Innenschaftes 3 verläuft, vom Hauptkörper 1 kommend, eine Optik 9, die, z. B. in üblicher Weise auswechselbar angeordnet ist. Ferner ist im Inneren des Innenschaftes 3 ein z.B. wie in Figur 10 der eingangs genannten Schrift ausgebildeter Elektrodenträger 10 angeordnet, der längsverschiebbar angeordnet ist und an seiner distalen Spitze eine Schneidschlinge 11 trägt.

Mit der Schneidschlinge 11 wird unter HF-Strombeaufschlagung rückwärts ziehend gegen die distale Schneidkante 12 des Schaftes gearbeitet. Der distale Endbereich ist daher aus hochwarmfestem und isolierendem Material als rohrförmiger Isolierkörper 13 ausgebildet.

Wie Figur 1 zeigt, ist der Isolierkörper 13 durch Verklebung oder dergleichen flächig mit dem Außenschaft 5 fest verbunden. Der gegenüber dem Außenschaft 5 und somit gegenüber dem Isolierkörper 13 abnehmbare Innenschaft 3 ist in seinem distalen Endbereich aufgeweitet und stößt, wie Figur 1 zeigt, gegen die proximale Kante 14 des Isolierkörpers 13.

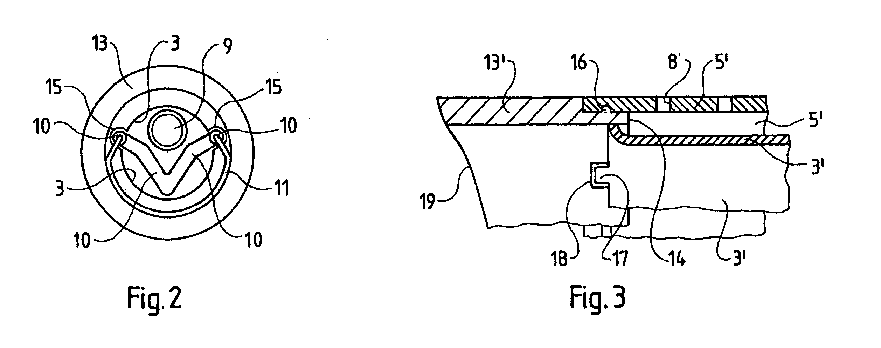

Wie Figur 2 zeigt, folgt die Schneidschlinge 11 eng anliegend einem Teil des Innendurchmessers des Isolierkörpers 13, um mit möglichst großem Durchmesser große Gewebeschnitte ausführen zu können. Der Elektrodenträger 10 bildet im distalen Endbereich die beiden in Figur 2 erkennbaren Gabelarme aus, die zur besseren Stabilität der Schneidschlinge 11 möglichst großen Abstand zueinander haben sollen. Hierzu weist der Innenschaft 3 die in Figur 2 erkennbaren Nuten 15 auf, die bei großlumigem Ringkanal zwischen Innenschaft 3 und Außenschaft 5 den Gabelarmen des Elektrodenträgers 13 einen großen Abstand ermöglichen.

In moderner Bauweise sind beim dargestellten Ausführungsbeispiel Innenschaft 3 und Außenschaft 5 gegeneinander drehbar gelagert, beispielsweise durch entsprechende Ausbildung der Kupplungsstücke 2, 4. Zu Einzelheiten dieser Konstruktionsweise wird auf die DE 4101472 C2 verwiesen. Werden bei der Ausführungsform der Figur 1 die Schäfte 3, 5 gegeneinander gedreht, so dreht sich der Außenschaft 5 gegenüber dem Innenschaft 3, welcher jedoch in üblicher Ausbildung feststehend gegenüber dem Hauptkörper 1 bleibt und somit auch drehfest gegenüber dem Elektrodenträger 10 und der Schneidschlinge 11. Die Schneidschlinge 11 dreht sich somit gegenüber dem am Außenschaft 5 befestigten Isolierkörper 13. Um unter allen Drehstellungen der Schneidschlinge 11 gegenüber dem Isolierkörper 13 eine konstante Schneidkante 12 zu gewährleisten, ist bei der Ausführungsform der Figur 1 der Isolierkörper 13 an seinem distalen Ende gerade abgeschnitten.

Figur 3 zeigt eine Ausführungsvariante der Figur 1, bei der nur die abweichenden Teile erläutert sind, für die die in den Figuren 1 und 2 verwendeten Bezugszeichen verwendet werden, versehen mit einem Beistrich. Die übrigen Teile entsprechen der Ausführungsform der Figuren 1 und 2.

Der Isolierkörper 13' ist bei dieser Konstruktionsvariante wiederum am Außenschaft 5' befestigt, jedoch nicht feststehend, sondern diesem gegenüber verdrehbar. Wie Figur 3 zeigt ist zur verliersicheren drehbaren Befestigung der dargestellte Flanscheingriff als Gleitlager mit haltendem Nut- und Federeingriff 16 ausgebildet.

Wie Figur 3 zeigt ist eine Drehkupplung zwischen Innenschaft 3' und Isolierkörper 13' vorgesehen. Diese ist schematisch angedeutet als Vorsprung 17 am distalen Rand des Innenschaftes 3', der in eine Ausnehmung 18 im Isolierkörper 13' greift. Es sind auch andere Ausgestaltungsformen der Drehkupplung möglich.

Bei der Ausführungsform der Figur 3 wird bei Verdrehung des Außenschaftes 5' gegenüber dem Innenschaft 3' der Isolierkörper 13' mit dem Innenschaft 3' gedreht, somit also auch mit dem Hauptkörper 1 und mit der Schneidschlinge 11. Diese steht somit immer drehfest gegenüber dem Isolierkörper 13', der daher, wie dargestellt, in der bekannten Schnabelform mit abgeschrägtem distalem Rand 19 ausgebildet sein kann.

Der Innenschaft 3' könnte gemäß Figur 1 ausgebildet sein und auch bei der Ausführungsform der Figur 3 gegen die proximale Kante 14 des Isolierkörpers 13' stoßen. Er ist hier jedoch in einer Ausführungsvariante mit seinem aufgeweiteten distalen Rand gegen die Innenfläche des stoßend ausgebildet.

Würde der Innenschaft 3', abweichend von der dargestellten Ausführungsform, mit seinem distalen Rand weiter distal liegen, also den Isolierkörper 13' weiter überlappen, dann könnten zusätzliche, nicht dargestellte Löcher im Isolierkörper 13' vorgesehen sein, um einen besseren Rückstromzugang zum Ringkanal zwischen Innenschaft 3' und Außenschaft 5' zu gewährleisten.

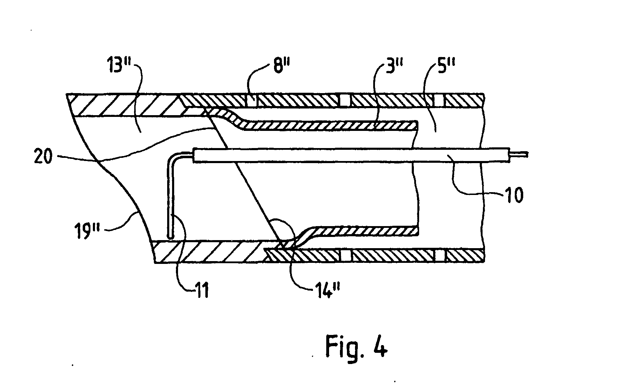

Wird im Hauptanwendungsfall ein Resektoskop durch die Urethra bis in den Bereich der Prostata verlegt, um dort mit der Schneidschlinge zu resizieren, so ist die gesamte Länge des Außenschafts bis in den unmittelbaren distalen Endbereich fest von der Urethra umschlossen. Die Löcher 8 im Außenschaft sind dann verlegt. Es muß daher dafür Sorge getragen werden, daß diese Löcher möglichst weit distal angeordnet sind. Hierfür kann eine weitere Konstruktionsvariante hilfreich sein, die in Figur 4 dargestellt ist. Es sind hier wiederum nach Möglichkeit gleiche Teile mit denselben Bezugszeichen versehen, ggf. mit zwei Beistrichen ergänzt.

Im Gegensatz zu den Ausführungsformen der Figuren 1 bis 3 ist bei dieser Ausführungsvariante der distale Rand 20 des Innenschaftes 3" abgeschrägt ausgebildet. In der Darstellung der Figur 4 liegt er im oberen Bereich weiter distal als im unteren Bereich. Das obere vorderste Loch 8" liegt somit weiter distal angeordnet als dies bei den Ausführungsformen der Figuren 1 bis 3, bei denen der distale Rand des Innenschaftes gerade angeordnet ist, möglich wäre. Hierdurch wird, also auch bei sehr weit nach distal umschlossenem Außenschaft noch eine Dauerspülung durch dieses Loch 8" gewährleistet.

Die in Figur 4 dargestellte Konstruktion entspricht ansonsten in ihren Ausführungsdetails der der Figur 1, bis auf die Tatsache, daß der distale Rand 19" des Isolierkörpers 13" schnabelartig abgeschrägt ist, wie bei der Konstruktion der Figur 3. Dabei liegen die abgeschrägten Ränder 20 und 19" im wesentlichen parallel. Die Abschrägung ist so gewählt, daß in dem Bereich in dem die Schneidschlinge 11 den Isolierkörper 13" berührt, der distale Rand 20 des Innenschaftes 3" weiter proximal liegt. Die Schneidschlinge 11 kann also, dem Isolierkörper 13" anliegend, soweit nach proximal zurückgezogen werden, daß Sie bereits auf Höhe des vordersten Lochs 8" liegt.

Wie Figur 4 zeigt, ist die Konstruktion hier entsprechend der der Figur 1 so gewählt, daß der distale Rand 20 des Innenschaftes 3" gegen die proximale Kante 14 " des Isolierkörpers 13" stößt. Es kann bei der abgeschrägten Konstruktionsweise der Figur 4 jedoch auch gemäß Figur 3 der distale Rand des Innenschaftes 3" von innen gegen die Innenwand des Isolierkörpers 13" stoßend ausgebildet sein. Dann kann der distale Rand des Innenschaftes 3" sogar noch etwas weiter nach distal verlegt werden und es könnte das vorderste Loch 8", das bei der Ausführungsform der Figur 4 den Außenschaft 5" durchsetzt, den Isolierkörper 13" durchsetzend angeordnet sein.

Es sind weitere nicht dargestellte Konstruktionsvarianten möglich. Es wurde bereits gemäß Figur 1 beschrieben, daß Außenschaft 5 und Innenschaft 3 abnehmbar am Hauptkörper 1 befestigt sein können. Zu Figur 3 wurde erwähnt, daß Innenschaft 3' und Schneidschlinge 11 zueinander drehfest stehen können, während der Außenschaft 5' beiden gegenüber drehbar gelagert ist.

In einer weiteren Ausführungsvariante könnte der Innenschaft fest am Hauptkörper 1 angeordnet sein und nur der Außenschaft abnehmbar und ggf. drehbar gelagert sein.

In einer weiteren nicht dargestellten Ausführungsvariante könnten beide Schäfte, also sowohl der Außenschaft als auch der Innenschaft, drehfest und ggf. abnehmbar am Hauptkörper 1 befestigt sein, während die Schneidschlinge 11 durch entsprechende drehbare Ausbildung des Schlingentransporteurs drehbar gegenüber dem Hauptkörper 1 gelagert ist.

## Patentansprüche

1. Urologisches Resektoskop mit einem im distalen Endbereich Öffnungen (8) aufweisenden Außenschaft (5, 5') und einem Innenschaft (3, 3"), die beide rohrförmig und aus Metall bestehend ausgebildet sind, die ferner im distalen Endbereich gegeneinander abgedichtet sind und die in ihrem proximalen Endbereich lösbar aneinander und an einem Hauptkörper (1) befestigt sind, mit einem den Innenschaft durchlaufenden, längsbewegbar angeordneten Elektrodenträger (10), der am distalen Ende eine HF-beaufschlagbare Schneidschlinge (11) trägt, mit proximal angeordneten Flüssigkeitsanschlüssen (7) zur Zufuhr von Flüssigkeit in den Innenschaft und zur Ableitung von Flüssigkeit aus dem Ringraum zwischen Innenschaft und Außenschaft, und mit einem distal am Außenschaft (5, 5') befestigten und gegen den Innenschaft (3, 3") anliegenden, rohrförmigen Isolierkörper (13, 13'), der denselben Außendurchmesser, jedoch einen kleineren Innendurchmesser als der distale Endbereich des Außenschaftes (5, 5') aufweist, **dadurch gekennzeichnet, dass** der Innenschaft (3) mit seinem im Durchmesser aufgeweiteten distalen Rand gegen den proximalen Rand (14) des Isolierkörpers stößt.

2. Resektoskop nach Anspruch 1, **dadurch gekennzeichnet, daß** die Schneidschlinge (11) in ihrer proximalen Endstellung distal vom distalen Ende des Innenschaftes (3, 3") angeordnet ist.

3. Resektoskop nach Anspruch 1, **dadurch gekennzeichnet, daß** der Innenschaft (3, 3") Längsnuten (15) zur Aufnahme der die Schneidschlinge (11) tragenden Gabelarme des Elektrodenträgers (10) aufweist.

4. Resektoskop nach Anspruch 1, **dadurch gekennzeichnet, daß** der Isolierkörper (13') drehbar am Außenschaft (5') befestigt und mit dem Innenschaft (3') drehgekoppelt (17, 18) ist.

5. Resektoskop nach Anspruch 1, **dadurch gekennzeichnet, daß** der distale Rand (20) des Innenschaftes (3") schräg zur Schaftachse verlaufend ausgebildet ist.

## Claims

1. Urological resectoscope comprising an outer stem (5, 5') having orifices (8) in its distal end region and an inner stem (3, 3'), wherein both stems are of tubular construction and are made from metal and furthermore are sealed against one another in the distal end region and in their proximal region are releasably fixed to one another and to a main body (1), further comprising an electrode support (10) which passes through the inner stem and is disposed so as to be longitudinally movable and bears on the distal end a cutting loop (11) to which an HF current can be applied, further comprising proximally disposed fluid connections (7) for feeding fluid into the inner stem and for drawing off fluid from the annular space between the inner and outer stems, and further comprising a tubular insulating body (13, 13') which is fixed distally on the outer shaft (5, 5') and abuts the inner shaft (3, 3") and which has the same external diameter as the distal end region of the outer stem (5, 5') but a smaller internal diameter than it, **characterised in that** the inner stem (3) butts with its diametrically widened distal edge against the proximal edge (14) of the insulating body.

2. Resectoscope as claimed in Claim 1, **characterised in that** the cutting loop (11) in its proximal end position is disposed distally from the distal end of the inner stem (3, 3').

3. Resectoscope as claimed in Claim 1, **characterised in that** the inner stem (3, 3') has longitudinal grooves (15) to receive the fork arms of the electrode support (10) which bear the cutting loop (11).

4. Resectoscope as claimed in Claim 1, **characterised in that** the insulating body (13') is rotatably fixed on the outer stem (5') and is coupled in rotation (17, 18) with the inner stem (3').

5. Resectoscope as claimed in Claim 1, **characterised in that** the distal edge (20) of the inner stem (3") is constructed so as to extend obliquely with respect to the axis of the stem.

## Revendications

1. Résectoscope urologique muni d'une gaine externe (5, 5') dotée dans sa section d'extrémité distale d'orifices (8) et une gaine interne (3, 3"), ces deux gaines étant de conformation tubulaire et en métal et étant, par ailleurs, isolées l'une par rapport à l'autre au niveau de la section d'extrémité distale et fixées au niveau de leur section d'extrémité proximale de façon amovible l'une à l'autre et à un corps principal (1), muni d'un porte-électrode (10) traversant la gaine interne agencé de façon longitudinalement mobile et portant à son extrémité distale une anse de résection (11) à laquelle peut être appliqué un courant HF, muni de raccords (7) pour liquides agencés du côté proximal en vue de l'adduction de liquide dans la gaine interne et de l'évacuation de liquide hors de l'espace annulaire entre la gaine interne et la gaine externe, et muni d'un corps isolant (13, 13') de forme tubulaire fixé à la partie distale de la gaine externe (5, 5') et adjacent à la gaine interne (3, 3"), ce corps ayant le même diamètre extérieur que la gaine externe (5, 5') mais un diamètre intérieur inférieur à celui de la section d'extrémité distale de la gaine externe (5, 5'), **caractérisé en ce que** la gaine interne (3) bute, avec son bord distal de diamètre évasé, contre le bord proximal (14) du corps isolant.

2. Résectoscope selon la revendication 1, **caractérisé en ce que** l'anse de résection (11) est, en fin de course proximale, agencée en position distale par rapport à l'extrémité distale de la gaine interne (3, 3").

3. Résectoscope selon la revendication 1, **caractérisé en ce que** la gaine interne (3, 3") présente des rainures longitudinales (15) pour le logement des branches de support de l'anse de résection (11) du porte-électrode (10).

4. Résectoscope selon la revendication 1, **caractérisé en ce que** le corps isolant (13') est fixé à la gaine externe (5') de façon à pouvoir pivoter et est couplé à la gaine interne (3') par un accouplement tournant (17, 18).

5. Résectoscope selon la revendication 1, **caractérisé en ce que** le bord distal (20) de la gaine interne (3") est de conformation oblique par rapport à l'axe de la gaine.
